# EUROPEAN PATENT APPLICATION

(11) **EP 1 588 682 A1**
(43) Date of publication of application: **26.10.2005**
(21) Application number: 05252495.6
(22) Date of filing: 21.04.2005
(51) Int. Cl.: A61F 13/02, A61F 15/00, B65D 33/20

(54) **Portable package for articles of personal use**

(30) Priority: 22.04.2004 BR 0401630
(71) Applicant: Johnson & Johnson Industrial Ltda., Sao Jose dos Campos, SP (BR)
(72) Inventor: Lima, Viviane Antao, Santana Sao Paulo (BR); Aledo, Eduardo Ceasar Andreo, 235 Sao Jose dos Campos (BR)
(74) Representative: Mercer, Christopher Paul

(57) **Abstract**

A portable package for articles of personal use, such as feminine absorbents, comprising a strip (1) containing at least one row of wraps (10) in the form of tubular "pillows" arranged in series, each wrap (10) involving at least one personal use article (2) and being formed by a front wall (11) and a rear wall (12) having longitudinal lateral edges (13) and transversal end edges (14). Each confronting transversal end edge (14) of each two adjacent wraps (10) are united to each other by a joining flap (20), so that the wraps (10) can be folded one over the other.

## Description

### Field of the Invention

The present invention refers to a portable package for containing articles of personal use, such as feminine absorbents, particularly internal or external absorbents, daily use protectors or body fluid absorbing products.

### Prior Art

There are well known from the art the packages in the form of small bags made of plastic film or other material, or also in the form of paper or paperboard boxes, involving a plurality of absorbents, each absorbent being preferentially and individually involved, generally in a condition in which it is folded one or more times by a wrap made of plastic film or paper, and in the form of a "pillow", having the longitudinal lateral edges mutually seated in superposition but not affixed to each other, and with the transversal end edges being sealed by any adequate process, such as heat, ultrasound, adhesive, etc.

These conventional packages of feminine absorbents or daily use protectors are adequate to be stored in fixed locations, defining a supply to be maintained at home or at work and from which the user can extract the pillow-shaped unit to be individually applied to the body, after the wrap is ruptured at the superposed longitudinal lateral edges.

These known packages generally contain a number of absorbents designed to fulfill the needs for more than one day, usually several days, or in situations requiring more frequent changes.

Thus, the present packages are not adequate to facilitate the user to carry, if necessary, a small number of absorbents considered sufficient to fulfill an immediate need or one which lasts only until the usual supply at home or at work can be accessed again. In the present state of the art, the users have to carry a package containing ten or more units of absorbents, which is an excessive quantity in relation to the immediate needs and usually causing problems of portability.

Besides the disadvantages mentioned above, the present packages containing a fixed and relatively large quantity of absorbents are not sufficiently versatile to comply with the sporadic needs, always requiring the formation of a minimum supply which can be excessive and even unjustifiable in many situations.

Another disadvantage of the known packages results from the fact of having the pillow-shaped wraps carried loosely inside the user's purse, allowing that the generally superposed edges which define the access opening to the interior of the wrap remain exposed to the interior of the purse and thus subject to friction with other objects therein and consequently to displacement or deformation, impairing the ideal closing condition and with their content being vulnerable to strange bodies and contaminants in contact with the packaged absorbents.

### Object of the Invention

As a function of the disadvantages mentioned above, it is a generic object of the present invention to provide a portable package for articles of personal use, such as feminine absorbents, particularly feminine absorbents or daily use protectors, which allows the user to carry the desired number of units which is adequate to her immediate needs, said units being protected, individually or jointly, in an arrangement which facilitates the portability and accessibility thereof in a purse or any other similar means.

### Summary of the Invention

In order to comply with the objects of the present invention, the portable package proposed herein comprises a strip made of any adequate material and generally presenting a flat tubular shape, containing at least one row of wraps in the form of tubular "pillows" arranged in series, each involving at least one feminine absorbent and being formed by a front wall and a rear wall having longitudinal lateral edges on each side of the row and transversal end edges united to each other. The transversal end edges of each two adjacent wraps of the same row are united to each other by a joining flap, in order to allow the wraps to be folded one over the other and eventually separated from each other.

The joining flap allows each wrap to be folded in such a way as to be seated on an adjacent wrap, which arrangement considerably facilitates the user to carry a row of wraps inside a purse or bag.

In the cases in which each wrap contains more than one absorbent, the longitudinal closing of each wrap is preferably made by simple superposition and gluing, or removably sealing two edges of one of the front or rear walls thereof, allowing the user to have access to the interior of the wrap to take an absorbent, closing said wrap again so that it can be easily carried inside a purse, maintaining the integrity of the absorbents still contained inside the wrap, without requiring the provision of additional external wraps for involving the whole wrap assembly.

### Brief Description of the Drawings

The invention will be described below, with reference to the enclosed drawings, given by way of example of some possible constructions of the invention and in which:
Figure 1 is a perspective view of a portable package object of the present invention formed by a strip containing a row with two wraps;
Figure 2 is a perspective view of the package of figure 1 with the wraps being folded one over the other, in the form of a case;
Figure 3 is a partial sectional view of the package illustrated in figure 1, said section being taken according to line III-III of said figure 1;
Figure 4 is a cross-sectional view of the package of figure 1, said section being taken according to line IV-IV of said figure 1;
Figure 5 is a perspective view of a second embodiment of the present package, also formed by a strip containing a row with three wraps;
Figure 6 is a perspective view of the package of figure 5, with the wraps being folded and seated one over the other, in the form of a case;
Figure 7 is a perspective view of a third embodiment of the present package, also formed by a strip containing, exemplarily, a row with three wraps;
Figure 8 is a sectional view of the package of figure 7, with the wraps folded one over the other;
Figure 9 is a perspective view of a fourth embodiment of the present package when formed by a strip containing, exemplarily, a row with an even number of wraps;
Figure 10 is a partially cut perspective view of the package of figure 9 when folded in the form of a case; and
Figure 11 is a view similar to that of figure 9, but illustrating the rear side of the present package.

### Disclosure of the Illustrated Embodiments

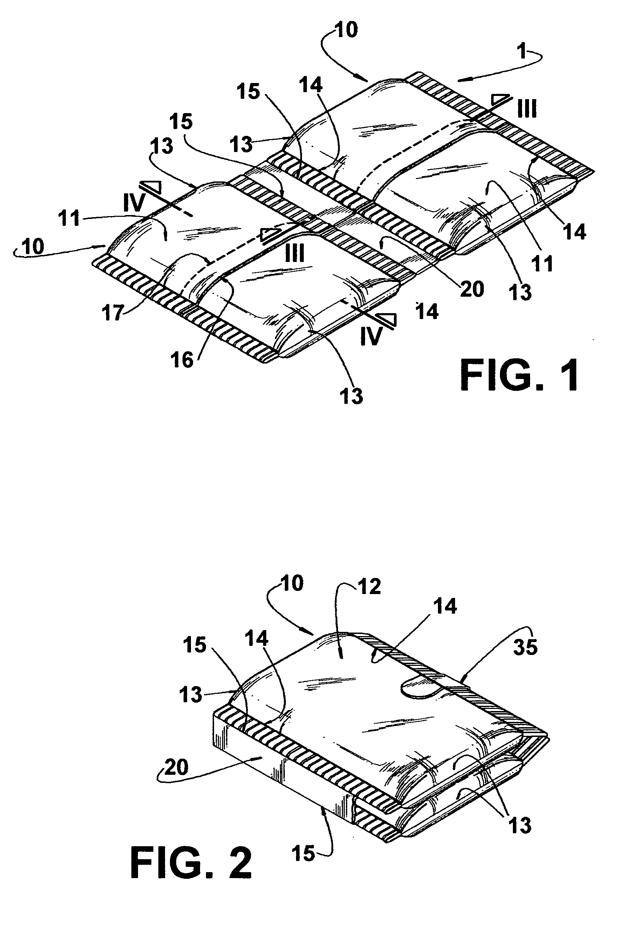

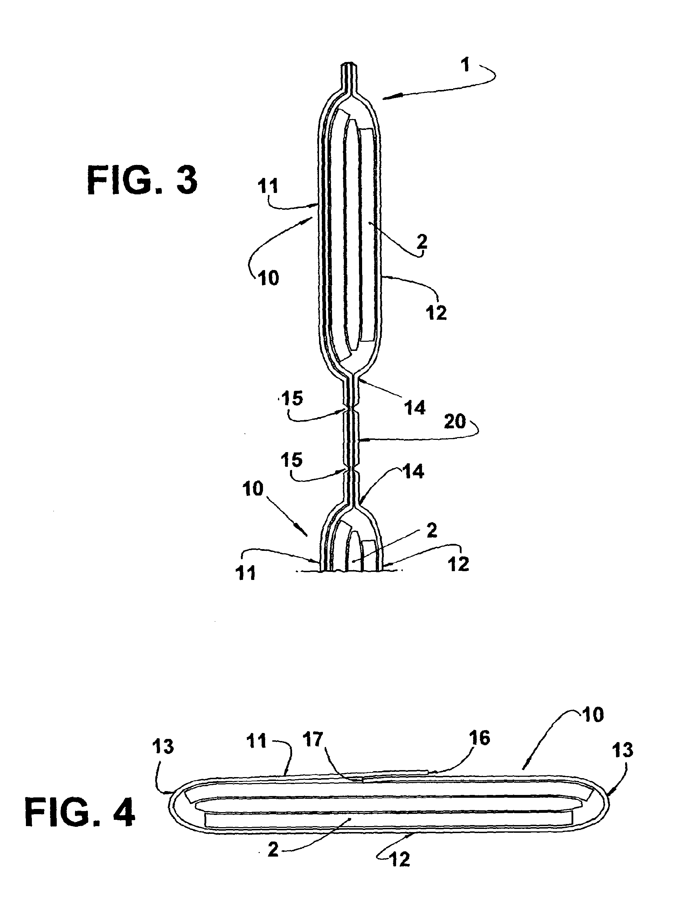

As illustrated in figures 1-4 of the enclosed drawings, the present portable package comprises a strip 1 generally presenting a flat tubular shape and which is made of at least one film of plastic material or paper, or any material adequate to the package of the present product. Each strip 1 contains at least one row with one or more wraps 10 shaped as tubular "pillows" and arranged in series, each wrap 10 involving at least one personal use article 2, usually a feminine absorbent or daily use protector, which is presented in the flat form or preferably folded one or more times, such as the construction illustrated in said figures 1-4.

Each wrap 10 of a row is formed by a front wall 11 and a rear wall 12, which present longitudinal lateral edges 13 on each side of the row and transversal end edges 14 united to each other by heat, ultrasound, etc. The confronting transversal end edges 14 of each two adjacent wraps 10 in the same row are connected to each other by a respective joining flap 20, which is usually united to the confronting transversal end edges 14 of the two adjacent wraps 10 by a respective fold line 15, around which the wraps 10 can be folded in order to occupy positions parallel to each other and orthogonal to the joining flaps 20, defining a case, as illustrated in figure 2.

It is possible to construct the package so that at least one of each two fold lines 15 adjacent to a joining flap 20 presents cutouts and/or weakened lines, in order to be manually ruptured by the user, when she desires to separate a wrap 10 from the row of wraps. This construction allows each wrap 10 to be easily detached from the other wraps 10 of the row, allowing the user to carry in her purse only the number of wraps needed for the intended use of the personal use articles 2 contained in each wrap.

As illustrated in the drawings, each row of wraps 10 of a strip 1 presents a certain number of wraps 10 to be determined as a function of the characteristics of commercialization and also as a function of the number of the personal use articles 2 to be contained inside each wrap.

As illustrated in the figures, particularly in figures 2, 5, 6, 7, 8, 9 and 10, the joining flaps 20 are made of the same material which forms the row of wraps, with the longitudinal edges 13 of the front wall 11 and rear wall 12 being likewise welded to each other.

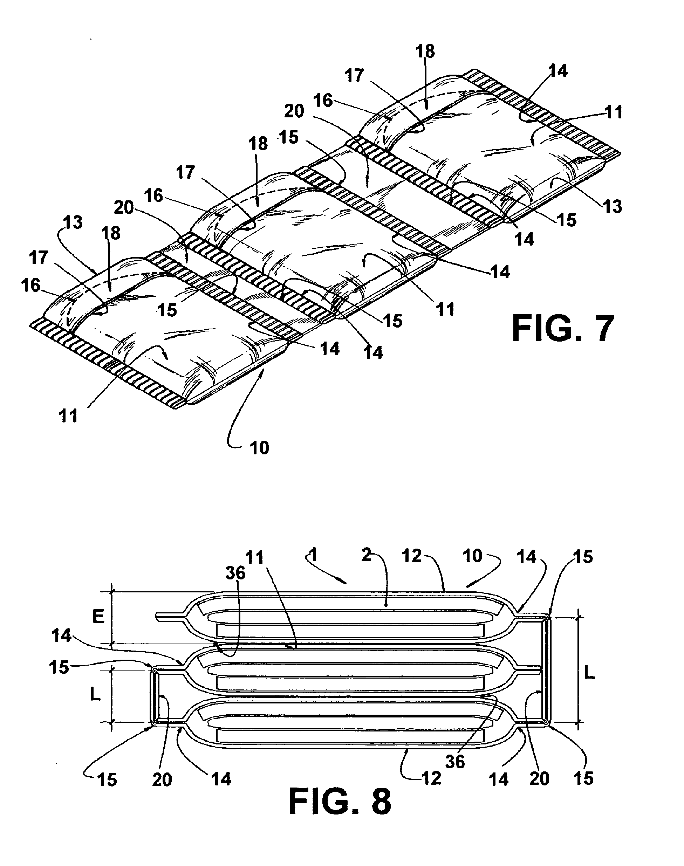

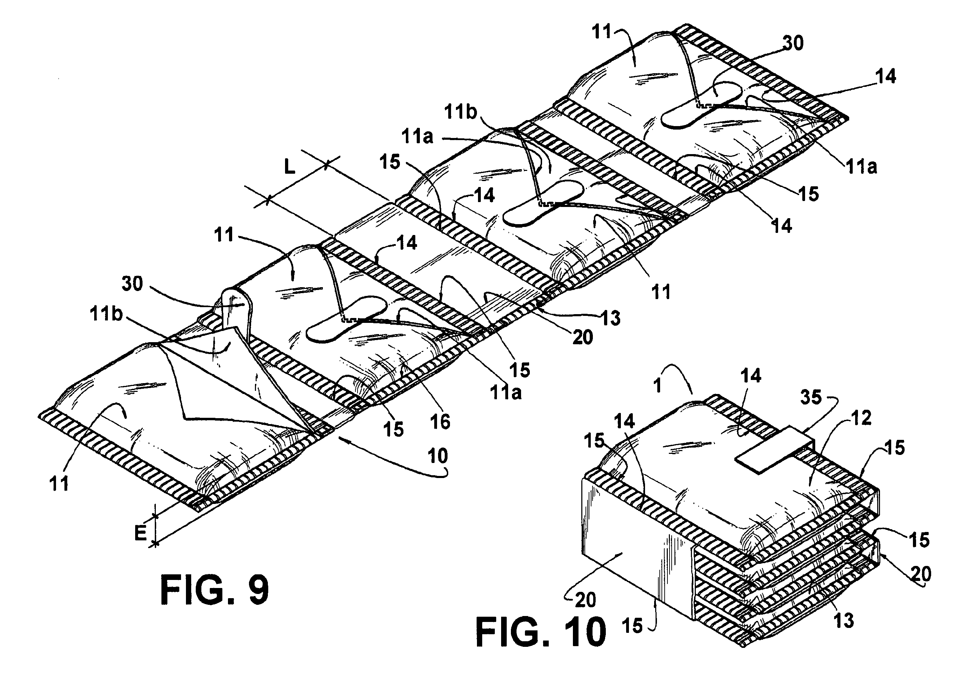

Each joining flap 20 presents, in the longitudinal direction of the row, an "L" extension (figures 8 and 9) which is dimensioned as a function of the folding pattern to be imparted to the wraps 10 (see figures 2, 6, 8 and 10), in order to approximately correspond to the sum of the thicknesses "E" of the wraps 10 disposed on planes parallel to each other and orthogonal and secant in relation to said joining flap 20, allowing the wraps to be conducted to a folded condition around the respective fold lines 15 in order to form a compact and reliable package in the form of a case which can be easily accommodated inside a purse or bag.

It should be understood that each strip 1 can be formed by two or more rows of wraps 10 disposed side by side, each row being separated from the immediately adjacent row by a longitudinal fold line (not illustrated), but which is constructed according to the same characteristics applied to the fold lines 15 that divide each consecutive two wraps 10 in a row.

In order to facilitate the accommodation and transportation of a row of wraps 10 inside a purse or bag, the fold lines 15 also define the fold lines around which the wraps 10 can be folded so as to be seated on the immediately adjacent wraps 10.

In the construction illustrated in figures 1-4 of the drawings, the wraps 10 have their front wall 11 and rear wall 12 defined by a single continuous film of a packaging material, presenting opposite longitudinal edges 16 and 17 which are trespassed in superposition and defined by the front wall 11 and rear wall 12 of the wraps 10 and releasably affixed to each other, in order to allow a number of opening and closing operations of the wrap 10 which is at least equal to the number of the personal use articles 2 contained therein. This type of construction is particularly adequate in the cases in which each wrap 10 contains two or more personal use articles 2. In these cases, after taking a personal use article 2 from a wrap 10 by opening the longitudinal edges 16, 17 of the front wall 11 and rear wall 12 of each wrap 10 and which are defined by the single continuous film of a packaging material, these same longitudinal edges 16, 17 can be mutually seated again so as to maintain the personal use articles 2 protected inside the respective wrap, until they are removed by the user. This type of construction makes each wrap 10 be transformed in a reclosable package to contain a plurality of personal use articles 2, greatly facilitating the transportation thereof in a condition in which the integrity of said personal use articles 2 remains guaranteed, while they are carried inside purses or bags.

The releasable fixation of the longitudinal edges 16, 17 of the front wall 11 and rear wall 12 of each wrap 10 can be achieved, such as by using an adequate adhesive to be applied to one of the confronting faces adjacent to one of said longitudinal edges 16, 17.

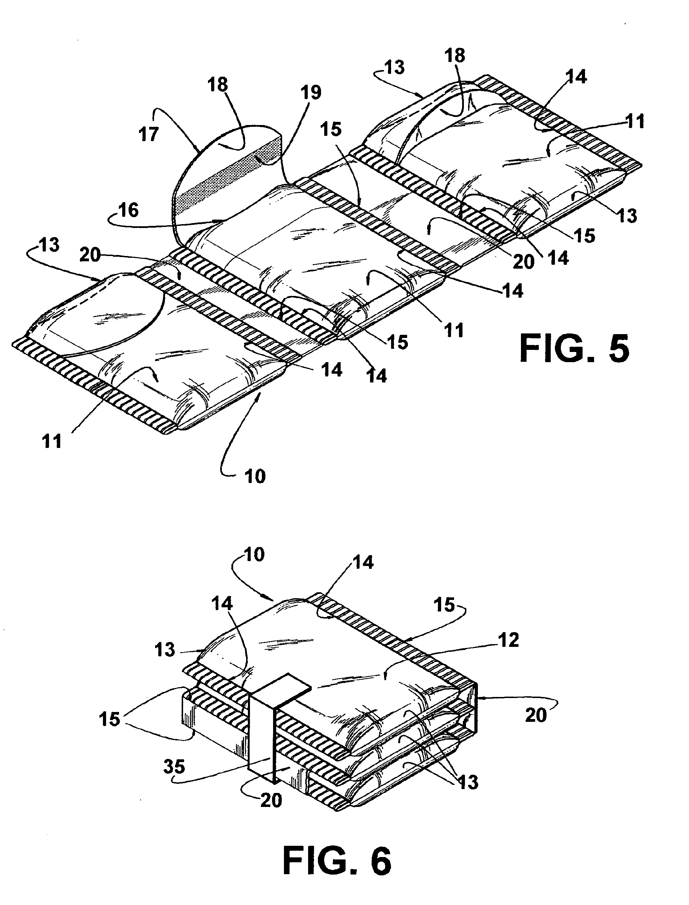

Figures 5 and 6 refer to a second embodiment of the invention, according to which each wrap 10 of one row of wraps presents the longitudinal edge 16 of the front wall 11 of the wrap, defined between the respective pair of transversal end edges 14, substantially coinciding, at least at the end portions thereof, with the adjacent longitudinal lateral edge 13 of the wrap 10, the longitudinal edge 17 of the rear wall 12 of the wrap being defined in a closing flap 18, which is dimensioned to be forwardly folded and seated, in superposition, onto a certain extension of the front wall 11 of the respective wrap 10, so as to maintain the personal use articles 2 protected inside the wrap 10.

As illustrated in figures 5 and 6, the closing flap 18 of the rear wall 12, as well as the front wall 11, can be provided, respectively, in their internal and/or external faces with a coating of adhesive 19, in order to guarantee a releasable and reclosable retention of the closing flap 18 on the front wall 11 of the wrap. It should be understood that, in this second embodiment illustrated in figures 5 and 6, the package could contain a larger number of wraps 10 separated from each other by respective joining flaps 20, as illustrated in figures 7, 8, 9, 10 and 11.

Figures 7 and 8 refer to a third embodiment of the invention, similar to that of figures 5 and 6, according to which each wrap 10 of a row of wraps presents the longitudinal edge 16 of the front wall 11 of the wrap, defined between the respective pair of transversal end edges 14, in the form of an arc of a circle medianly tangent to the alignment of the adjacent longitudinal lateral edge 13 of the wrap 10, the longitudinal edge 17 of the rear wall 12 of the wrap being defined in a closing flap 18 dimensioned to be forwardly folded and seated, in superposition, onto a certain extension of the front wall 11 of the respective wrap 10, in order to maintain the personal use articles 2 protected inside the wrap.

In this third wrap embodiment usually utilized to contain multiple personal use articles 2 which are not folded, the degree of superposition of the closing flap 18 in relation to the front wall 11 of the wrap 10 is sufficiently large to make optional the application of adhesive on the internal face of the closing flap 18 and/or on the external of the front wall 11.

Figures 9, 10 and 11 refer to a fourth embodiment of the invention, according to which each wrap 10 of a row of wraps presents the longitudinal edges 16, 17 of the front wall 11 and rear wall 12 welded to each other by heat, ultrasound or any other adequate process, defining the respective longitudinal lateral edge 13 of the wraps 10 of the row.

In this construction, the four limiting edges of each wrap 10 are closed, and the access to the interior of each wrap 10 is obtained by a generally "V" shaped cutout 11a produced in the front wall 11 of each wrap 10 and which forms a closing portion 11b, having an edge defined by a transversal end edge 14 of the wrap 10 and the opposite free edge defined by the cutout 11a produced in the front wall 11 of the wrap 10. The closing portion 11b can thus be manually displaced by the user between a closed position, in which it remains with its free edge leveled with the remaining part of the front wall 11 in the region of the cutout 11a, and a raised open position, in which it releases the access to the inside of the wrap 10.

In order to assure the retention of the closing portion 11b in the closed position, there can be provided a lug 30, which is detachably glued to the front wall 11 of each wrap 10, transversally over the cutout 11a so as to maintain the closing portion 11b in the closed condition.

It should be understood that the lug 30 could be further applied to the closing flaps 18 of the wraps 10 of the embodiments illustrated in figures 5-6 and 7-8 to make the closure of the wraps 10 more efficient.

In the embodiment illustrated in figures 9-11, the package is constituted by an even number of wraps 10, usually four, the wraps 10 being separated from each other by respective joining flaps 20 formed by the film that forms the wraps 10 and presenting an extension "L" which corresponds, approximately, to the sum of the thicknesses "E" of the wraps 10 which, when folded, are disposed on planes parallel to each other and orthogonal and secant to a respective joining flap 20. In general, it can be said that the extension "L" of each joining flap 20 is at least equal to the sum of the thicknesses "E" of the "n-1" wraps 10 that are orthogonal and secant to said joining flap 20 and not superior to the sum of the thicknesses "E" of said "n" wraps 10.

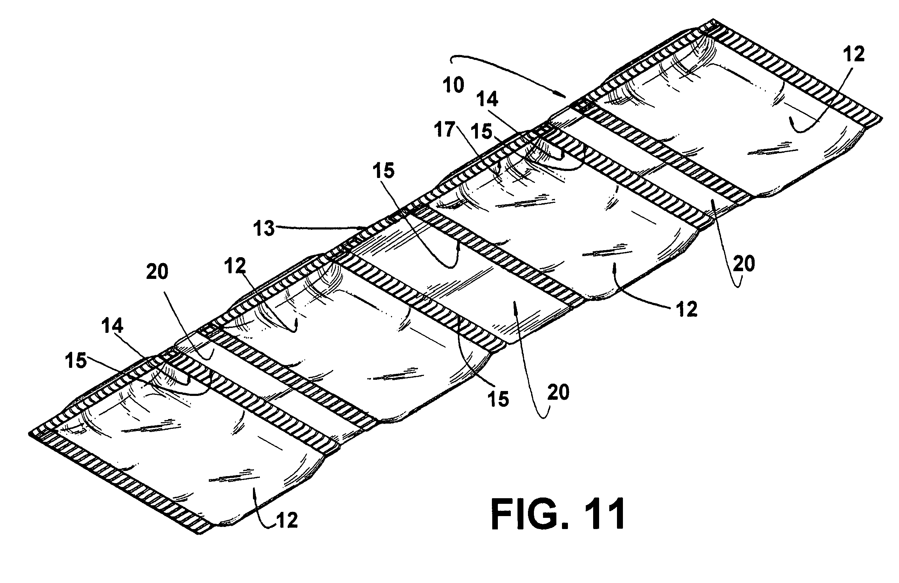

With the described construction, the outermost wraps 10 can be folded over the innermost wraps 10, until the wraps 10 occupy positions that are parallel and adjacent to each other and orthogonal and secant to the respective joining flaps 20, forming a kind of case, as illustrated in figure 10.

In order to maintain the wraps 10 in the folded condition illustrated in figures 2, 6, 8 and 10, there can be provided a closing flap 35 for connecting, in a selectively releasable manner, the transversal end edges 14 of the two outermost wraps 10 of the case to be formed. It should be understood that the closing flap 35 could be replaced by a drop of a "hot-melt" adhesive 36 applied to the mutually seated internal faces of the wraps 10 when in the folded condition, as illustrated in figure 8, or by any other fixation means.

While only one way of carrying out the invention has been illustrated herein, it should be understood that changes in the form and arrangement of the different parts could be made, without departing from the constructive concept defined in the appended claims.

## Claims

1. A portable package for articles of personal use, such as feminine absorbents, **characterized in that** it comprises a strip (1) containing at least one row of wraps (10) in the form of tubular "pillows" arranged in series, each wrap (10) involving at least one personal use article (2) and being formed by a front wall (11) and a rear wall (12) having longitudinal lateral edges (13) on each side of the row and transversal end edges (14) united to each other, the confronting transversal end edges (14) of each two adjacent wraps (10) of the same row being united to each other by a joining flap (20), in order to allow the wraps (10) to be folded one over the other and eventually separated from each other.

2. The package as set forth in claim 1, **characterized in that** a joining flap (20) is united to each of the confronting transversal end edges (14) of two adjacent wraps (10) by a respective fold line (15) around which the wraps (10) can be folded to occupy positions parallel to each other and orthogonal to the joining flaps (20).

3. The package as set forth in claim 2, **characterized in that** at least one of each two fold lines (15) adjacent to a joining flap (20) are cutout and/or weakened to be manually ruptured by the user when separating a wrap (10) from the row of wraps.

4. The package as set forth in claim 2, **characterized in that** the joining flap (20) has, in the longitudinal direction of the row, an extension (L), which is dimensioned as a function of the folding pattern to be imparted to the wraps (10), in order to correspond, approximately, to the sum of the thicknesses (E) of the wraps (10) disposed on planes parallel to each other and orthogonal and secant in relation to said joining flap (20).

5. The package as set forth in claim 4, **characterized in that** the extension (L) of each joining flap (20) is at least approximately equal to the sum of the thicknesses (E) of the "n-1" wraps (10) disposed on planes orthogonal and secant to said joining flap (20) and not superior than the sum of the thicknesses (E) of said "n" wraps (10).

6. The package as set forth in claim 4, **characterized in that** it comprises a closing flap (35) uniting, in a selectively released manner, the two external wraps (10) of a plurality of wraps folded one over the other.

7. The package as set forth in claim 4, **characterized in that** it comprises a drop of "hot-melt" adhesive (36) applied on the mutually seated internal faces of the wraps (10) when in the folded condition.

8. The package as set forth in claim 1, **characterized in that** the personal use articles (2) are arranged in a folded condition inside each respective wrap (10).

9. The package as set forth in claim 1, **characterized in that** the wraps (10) have their front wall (11) and rear wall (12) defined by a continuous film of packaging material, the front wall (11) and the rear wall (12) of each wrap (10) presenting longitudinal edges (16, 17) which are trespassed in superposition and releasably affixed to each other, in order to allow a number of opening and closing operations of the wraps (10) at least equal to the number of personal use articles (2) contained therein.

10. The package as set forth in claim 9, **characterized in that** the releasable fixation between the two longitudinal edges (16, 17) of the front wall (11) and rear wall (12) of each wrap (10) is obtained by the application of an adhesive (19) to at least one of the confronting and superposed faces of the front wall (11) and rear wall (12) of each wrap (10).

11. The package as set forth in claim 9, **characterized in that** the longitudinal edge (16) of the front wall (11) of a wrap (10), defined between the respective pair of transversal end edges (14), substantially coincides, at least in the external portions thereof, with the adjacent longitudinal lateral edge (13) of the wrap (10), the longitudinal edge (17) of the rear wall (12) of the wrap (10) being defined in a closing flap (18) forwardly folded and seated, in superposition, on a certain extension of the front wall (11) of the same wrap (10).

12. The package as set forth in claim 9, **characterized in that** the longitudinal edge (16) of the front wall (11) of a wrap (10), defined between the respective pair of transversal end edges (14), has the form of an arc of a circle medianly tangent to the alignment of the adjacent longitudinal lateral edge (13) of the wrap (10), the longitudinal edge (17) of the rear wall (12) of the wrap (10) being defined in a closing flap (18), which is dimensioned to be forwardly folded and seated, in superposition, over a certain extension of the front wall (11) of the wrap (10).

13. The package as set forth in any one of the claims 11 or 12, **characterized in that** at least one of the parts defined by the closing flap (18) and the front wall (11) is provided, on its face turned to the other part, with a drop of adhesive (19) so as to provide a releasable and reclosable retention of the closing flap (18) on the front wall (11) of the wrap (10).

14. The package as set forth in any one of the claims 11 or 12, **characterized in that** it comprises a lug (30) which is detachably glued to the closing flap (18) and to the adjacent portion of the front wall (1) of the wrap (10), in order to maintain said closing flap (18) in the seated superposed condition on part of said front wall (11).

15. The package as set forth in claim 1, **characterized in that** the front wall (11) and the rear wall (12) of each wrap (10) present, along one of the longitudinal lateral edges (13) of the row, respective longitudinal edges (16, 17) welded to each other, the front wall (11) being provided with a cutout (11a) defining, with a transversal end edge (14) of the wrap (10), a closing portion (11b) which is manually displaced between a closed position, in which it remains with its free edge defined by the cutout (11a), leveled with the remainder of the front wall (11) in the region of said cutout (11a), and a raised opened position, in which it liberates the access to the interior of the wrap (10).

16. The package as set forth in claim 15, **characterized in that** it comprises a lug (30) which is detachably glued to the front wall (11) of each wrap (10), transversally on the cutout (11a), in order to maintain the closing portion (11b) in the closed condition.
